(19) Europäisches Patentamt
European Patent Office
Office européen des brevets

(11) **EP 4 148 140 A1**

(12) **EUROPEAN PATENT APPLICATION**
published in accordance with Art. 153(4) EPC

(43) Date of publication:
**15.03.2023 Bulletin 2023/11**

(21) Application number: **21872174.4**

(22) Date of filing: **09.09.2021**

(51) International Patent Classification (IPC):
**C12Q 1/06** *(2006.01)*        **G01N 33/53** *(2006.01)*

(52) Cooperative Patent Classification (CPC):
**C12Q 1/06; G01N 33/53**

(86) International application number:
**PCT/JP2021/033099**

(87) International publication number:
**WO 2022/065043 (31.03.2022 Gazette 2022/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.09.2020   JP 2020159896**

(71) Applicant: **JVCKenwood Corporation
Yokohama-shi, Kanagawa 2210022 (JP)**

(72) Inventor: **ONO Masayuki
Yokohama-shi, Kanagawa 221-0022 (JP)**

(74) Representative: **Klang, Alexander H. et al
Wagner & Geyer Partnerschaft mbB
Patent- und Rechtsanwälte
Gewürzmühlstrasse 5
80538 München (DE)**

(54) **METHOD AND KIT FOR EVALUATING CONDITION OF CELL**

(57)    A method for evaluating a condition of cells including: Step (A) of measuring first exosomes having a first exosome membrane protein and second exosomes having a second exosome membrane protein in a culture supernatant of the cells; Step (B) of calculating the ratio of the second exosomes to the first exosomes; and Step (C) of determining the condition of the cells using the ratio as an index.

**Description**

[Technical Field]

The present invention relates to a method and a kit for evaluating a condition of cells.

Priority is claimed on Japanese Patent Application No. 2020-159896, filed September 24, 2020, the content of which is incorporated herein by reference.

[Background Art]

Currently, various cells are handled in various fields such as the regenerative medical field and disease research field. For example, in the regenerative medical field, stem cells are cultured, grown to a certain amount, and are then differentiated into desired cells and used for transplantation into humans. In addition, in use of cells in the disease research field, disease cells or therapeutic cells are cultured, and the properties and characteristics of the cells are evaluated.

In any field, it is necessary to evaluate the condition of cultured cells to use the cells. Currently, the evaluation of the condition of cells is performed based on shape evaluation through a microscopic observation and whether or not there is any abnormality in a culture process itself. However, it cannot be stated that the condition of cells can be sufficiently evaluated. In addition, cells collected for a microscopic observation cannot be used for subsequent processes. Furthermore, since most of cells adhere to a scaffold material such as a dish, the collection operation may affect other cultured cells.

In recent years, a method has been proposed for evaluating the condition of differentiation of cells by analyzing miRNA extracellularly released by cells (Patent Literature 1). However, miRNA is susceptible to enzymatic degradation, and its measurement operation is complicated such that time is taken for the measurement. In addition, since it is necessary to perform amplification through PCR to detect miRNA, even a slight error often poses a serious problem.

[Citation List]

[Patent Literature]

[Patent Literature 1]
Japanese Unexamined Patent Application, First Publication No. 2018-113924

[Summary of Invention]

[Technical Problem]

In order to use cells in the regenerative medical field or the disease research field, it is necessary to always prepare cells under the same conditions. In order to avoid the influence of the cell collection operation on cultured cells, technology that can easily and accurately evaluate the condition of cells without directly collecting cells is required.

Therefore, an object of the present invention is to provide a method and a kit for evaluating a condition of cells, the method and the kit enabling easy evaluation of the condition of cells using a culture supernatant of the cells.

[Solution to Problem]

The present invention has the following aspects.

[1] A method for evaluating a condition of cells including: Step (A) of measuring first exosomes having a first exosome membrane protein and second exosomes having a second exosome membrane protein in a culture supernatant of the cells; Step (B) of calculating a ratio of the second exosomes to the first exosomes; and Step (C) of determining the condition of the cells using the ratio as an index.

[2] The method for evaluating a condition of cells according to [1], in which Step (A) is a step using a first antibody which has a specific binding activity for the first exosome membrane protein and to which a first labeled substance is bound, a second antibody which has a specific binding activity for the second exosome membrane protein and to which a second labeled substance is bound, and a third antibody which has a specific binding activity for a third exosome membrane protein contained in at least some of the first exosomes and at least some of the second exosomes and is immobilized on a solid-phase carrier, and Step (A) includes Step (i) of bringing the culture supernatant into contact with the third antibody immobilized on the solid-phase carrier, Step (ii) of bringing the first antibody and the second antibody into contact with the solid-phase carrier after Step (i), and Step (iii) of measuring the first labeled substance and the second labeled substance captured on the solid-phase carrier after Step (ii).

[3] A method for evaluating a condition of cells, including: Step (a) of measuring first exosomes having a first exosome membrane protein and second exosomes having a second exosome membrane protein in a culture supernatant of the cells at a time (t1); Step (b) of calculating a ratio (t1) of the second exosomes to the first exosomes at the time (t1); Step (c) of measuring the first exosomes and the second exosomes in the culture supernatant of the cells at a time (t2); Step (d) of calculating a ratio (t2) of the second exosomes to the first exosomes at the time (t2); and Step (e) of determining the condition of the cells based on a comparison result of the ratio (t1) and the ratio (t2).

[4] The method for evaluating a condition of cells according to [3], in which Step (a) and Step (c) are steps using a first antibody which has a specific binding activity for the first exosome membrane protein and to which a first labeled substance is bound, a second antibody which has a specific binding activity for the second exosome membrane protein and to which a second labeled substance is bound, and a third antibody which has a specific binding activity for a third exosome membrane protein contained in at least some of the first exosomes and at least some of the second exosomes and is immobilized on a solid-phase carrier, and Step (a) and Step (c) include Step (i) of bringing the culture supernatant into contact with the third antibody immobilized on the solid-phase carrier, Step (ii) of bringing the first antibody and the second antibody into contact with the solid-phase carrier after Step (i), and Step (iii) of measuring the first labeled substance and the second labeled substance captured on the solid-phase carrier after Step (ii).

[5] A kit for evaluating a condition of cells, including: a first antibody which has a specific binding activity for a first exosome membrane protein and to which a first labeled substance is bound, a second antibody which has a specific binding activity for a second exosome membrane protein and to which a second labeled substance is bound, and a third antibody which has a specific binding activity for a third exosome membrane protein and is immobilized on a solid-phase carrier, in which at least some first exosomes having the first exosome membrane protein and at least some second exosomes having the second exosome membrane protein have the third exosome membrane protein.

[Advantageous Effects of Invention]

According to the present invention, there is provided a method and a kit for evaluating a condition of cells, the method and the kit enabling easy evaluation of the condition of cells using a culture supernatant of the cells.

[Brief Description of Drawings]

Fig. 1A shows an example of a solid-phase carrier used in a method for evaluating a condition of cells according to one embodiment of the present invention.
Fig. 1B is a view illustrating an example of Step (i) in Step (A) of a method for evaluating a condition of cells according to one embodiment of the present invention.
Fig. 1C is a view illustrating an example of Step (ii) in Step (A) of a method for evaluating a condition of cells according to one embodiment of the present invention.

Fig. 2A is a schematic diagram illustrating an example of an exosome capture unit provided with a solid-phase carrier for use in an exosome counter.
Fig. 2B is a schematic cross-sectional view of the exosome capture unit taken along cut line A-A of Fig. 2A.
Fig. 2C is a schematic cross-sectional view for illustrating that a cartridge 52 of the exosome capture unit of Fig. 2A is removable.
Fig. 3A is a schematic cross-sectional view of the exosome unit taken along cut line B-B of Fig. 2A.
Fig. 3B is an enlarged cross-sectional view for illustrating the dimensional shapes of concave portions and convex portions of the exosome unit of Fig. 2A.
Fig. 4 shows results examining a relationship between a condition of cells and the ratio of CD63-positive exosomes to CD9-positive exosomes.

[Description of Embodiments]

Hereinafter, an embodiment of the present invention will be described in detail with reference to the drawings according to circumstances. In the drawings, the same or corresponding portions will be denoted by the same or corresponding reference numerals, and description thereof will not be repeated. The dimensional ratios in each drawing are exaggerated for explanation and do not necessarily coincide with the actual dimensional ratios.

"Antibodies" mean immunoglobulins having an antigen-binding activity. It is unnecessary for antibodies to be intact antibodies as long as these have an antigen-binding activity, and the antibodies may be antigen-binding fragments. In the present specification, the term "antibodies" includes antigen-binding fragments. The "antigen-binding fragments" are polypeptides which contain a part of an antibody and maintain an antigen-binding property of the original antibody. The antigen-binding fragments preferably include all six complementarity determining regions (CDRs) of an original antibody. That is, the antigen-binding fragments preferably include all of CDR1, CDR2, and CDR3 of a heavy chain variable region and CDR1, CDR2, and CDR3 of a light chain variable region. Examples of antigen-binding fragments include Fab, Fab', $F(ab')_2$, a variable region fragment (Fv), a disulfide bond Fv, a single-stranded chain Fv (scFv), and $sc(Fv)_2$.

Antibodies may be derived from any organisms. Examples of organisms from which antibodies are derived include, but are not limited to, mammals (such as humans, mice, rats, rabbits, horses, cows, pigs, monkeys, and dogs) and birds (chickens and ostriches).

Antibodies may be any class and subclass of immunoglobulins. In addition, antibodies may be monoclonal antibodies or polyclonal antibodies, but monoclonal antibodies are preferable.

Antibodies can be produced through well-known methods such as an immunization method, a hybridoma method, and a phase display method.

"Having a specific binding activity" means having a high binding activity for a target substance but having almost no binding activity for other substances. For example, antibodies having a specific binding activity for a first exosome membrane protein have a high binding activity for the first exosome membrane protein but have almost no binding activity for other substances. Accordingly, the antibodies bind only to first exosomes and hardly bind to other substances. The same applies to antibodies having a specific binding activity for a second exosome membrane protein and antibodies having a specific binding activity for a third exosome membrane protein.

The term "comprise" means that constituent elements in addition to target constituent elements are included. The term "consist of" means that constituent elements in addition to target constituent elements are not included. The term "consist essentially of' means that constituent elements in addition to target constituent elements are not included in an aspect exhibiting a special function (such as an aspect in which the effect of the invention is completely lost). In a case where the term "comprise" is mentioned in the present specification, the aspects of "consist of" and "consist essentially of" are also included.

Proteins (including antibodies) and cells may be isolated. Being "isolated" means being separated from a natural state. Proteins and cells mentioned in the present specification may be isolated proteins and isolated cells.

<Method for Evaluating Condition of Cells>

[First Embodiment]

In one embodiment, the present invention provides a method for evaluating a condition of cells. The method for evaluating a condition of cells of the present embodiment includes: Step (A) of measuring first exosomes having a first exosome membrane protein and second exosomes having a second exosome membrane protein in a culture supernatant of the cells; Step (B) of calculating the ratio of the second exosomes to the first exosomes; and Step (C) of determining the condition of the cells using the ratio as an index.

In the evaluation method of the present embodiment, the ratio of second exosomes having a second exosome membrane protein to first exosomes having a first exosome membrane protein in a culture supernatant of cells is acquired, and the condition of the cells is evaluated using the ratio as an index. Exosomes are membrane vesicles surrounded by a lipid bilayer membrane having a diameter of 50 to 150 nm, and intraluminal membrane vesicles germinating inward into the endosomal lumen are released extracellularly. Exosomes reflect properties of cells from which these are derived, and have various proteins on exosome membranes. Cells do not always release exosomes with constant properties, and exosomes reflect the condition of cells at the time of release. The evaluation method of the present embodiment is based on the knowledge that the ratio between the two kinds of exosomes having specific kinds of membrane proteins in a culture supernatant reflects the condition of cultured cells.

The "condition of cells" evaluated through the evaluation method of the present embodiment is based on characteristics exhibited by most of target cells when the target cells are cultured using a medium normally used for culturing the target cells under standard culture conditions. That is, an "abnormal state of cells" means that characteristics of cultured cells have deviated from the standard characteristics. In addition, a "normal state of cells" means that characteristics of cultured cells have not deviated from the standard characteristics. Examples of characteristics of cells include, but are not limited to, the form, the size, the adhesion state, the suspension state, the aggregation state, the metabolic activity, the gene expression profile, and the protein profile of cells. According to the evaluation method of the present embodiment, it is possible to determine the condition of cells without directly observing or analyzing the cells.

(Cells)

The evaluation method of the present embodiment is a method for evaluating the condition of cultured cells. Cells to be evaluated are not particularly limited as long as they are cells that can be cultured in vitro, and arbitrary cells can be used. Cells are preferably eukaryotic cells. Organisms from which cells are derived are not particularly limited, and may be animal cells or plant cells, but animal cells are preferable. Examples of animal cells include, but are not limited to, cells of mammals, birds, reptiles, amphibians, fishes, and arthropods. Cells are preferably mammalian cells, and examples of mammals include, but are not limited to, humans, non-human primates (such as monkeys, chimpanzees, and gorillas), rodents (such as mice, hamsters, and rats), rabbits, dogs, cats, cows, goats, sheep, and horses.

The types of cells are not particularly limited, and any types of cells can be used. Cells may be somatic cells or germ cells. Examples of cells include, but are not limited to, pluripotent stem cells such as embryonic stem cells (ES cells) and induced pluripotent stem cells (iPS cells); somatic stem cells such as hematopoietic stem cells, adipose stem cells, mesenchymal stem cells, neural stem cells, intestinal epithelial stem cells, and epidermal stem cells; immune system cells such as dendritic cells, macrophages, lymphocytes (such as T cells, B cells, and natural killer cells); various tissue cells such as osteocytes, osteoblasts, adipocytes, chondrocytes, skin cells, nerve cells, muscle cells, fibroblasts, liver cells, and their progenitor cells; and cancer cells and cancer stem cells. In addition, cells may be cells induced to differentiate from pluripotent stem cells or somatic stem cells, modified cells modified through a gene recombination technique or the like, or established cells.

(Culture of Cells)

A method for culturing cells is not particularly limited, and a well-known method can be used depending on types of cells. Media used for culture are liquid media, and well-known media can be selected depending on types of cells. Medium components necessary for proliferation and maintenance of cells can be appropriately selected depending on the types of the cells. Examples of medium components include, but are not limited to, saccharides, organic acids, amino acids, proteins, polysaccharides, fatty acids, inorganic salts, vitamins, trace elements, growth factors, cytokines, antibiotics, antioxidants, and buffer agents.

Media may be produced by adding components such as serum and serum substitutes to well-known basal media. The "basal media" are media containing the minimum components necessary for growth of cells to be cultured, and various types are known. Examples of basal media of animal cells include IMDM medium, Medium-199 medium, Eagle's Minimum Essential Medium (EMEM) medium, αMEM medium, Dulbecco's Modified Eagle's Medium (DMEM) medium, Ham's F12 medium, RPMI 1640 medium, Fischer's medium, MCDB 201 medium, McCoy's 5A medium, a well-known medium for stem cells, and a mixed medium thereof.

Examples of serum added to a basal medium include, but are not limited to, human serum, fetal bovine serum (FBS), bovine serum, calf serum, goat serum, horse serum, pig serum, sheep serum, rabbit serum, and rat serum. In addition, one or more kinds of serum substitutes selected from albumin, transferrin, fatty acids, insulin, sodium selenite, cholesterol, collagen precursors, trace elements, 2-mercaptoethanol, and 3'-thiolglycerol may be used.

The cell culture conditions can be appropriately selected depending on types of cells. Examples of animal cell culture conditions include a temperature of 30°C to 37°C, a $CO_2$ concentration of 2% to 7%, and an $O_2$ concentration of 0.1% to 21%. Cells may be appropriately subcultured depending on cell growth conditions.

(Step (A))

Step (A) is a step of measuring first exosomes having a first exosome membrane protein and second exosomes having a second exosome membrane protein in a culture supernatant of the cells.

"Exosome membrane proteins" are proteins present in an exosome membrane. Exosome membrane proteins may be pan-exosome membrane proteins or may be exosome membrane proteins which only some kinds of exosomes specifically have. "Pan-exosome membrane proteins" refer to membrane proteins ubiquitously possessed by exosomes. Being "ubiquitously possessed by exosomes" means being possessed by a wide variety of exosomes. Examples of pan-exosome membrane proteins include CD9, CD63, and CD81.

A first exosome is an exosome having a first exosome membrane protein and a second exosome is an exosome having a second exosome membrane protein. The first exosome membrane protein and the second exosome membrane protein are exosome membrane proteins that are different from each other. However, the first exosome and the second exosome may partially overlap each other. That is, a part of the first exosome may have the second exosome membrane protein in addition to the first exosome membrane protein. That is, a part of the second exosome may have the first exosome membrane protein in addition to the second exosome membrane protein.

Examples of combinations of the first exosome membrane protein and the second exosome membrane protein include a combination of pan-exosome membrane proteins (for example, CD9 and CD63, CD9 and CD81, and CD63 and CD81), a combination of a pan-exosome membrane protein and a specific exosome membrane protein, and a combination of specific exosome membrane proteins. "Specific exosome membrane proteins" are exosome membrane proteins other than pan-exosome membrane proteins, and are membrane proteins expressed only in some exosomes. As an example, the first exosome membrane protein is CD9, and the second exosome membrane protein is CD63.

A "culture supernatant" is a liquid-phase fraction of a culture solution in which cells are cultured. It can also be said that a culture supernatant is a portion of a culture solution of cells from which the cells are removed. In a case where cells adhere to or settle on the bottom surface of a culture container, a culture supernatant can be obtained by collecting a supernatant portion of a culture solution. In a case where cells are suspended in a culture solution, a sample may be collected from the top of the culture solution, and a solid content may be removed through centrifugation, filtration, or the like.

A culture supernatant collected from a culture solution may be used as it is, or a culture supernatant obtained by separating exosome fractions through ultracentrifugation or the like may be used.

The ratio of second exosomes to first exosomes in a culture supernatant reflects the condition of cultured cells when the culture supernatant is collected. For this reason, the culture supernatant can be appropriately collected when the condition of cultured cells is to be evaluated, and can be used for measuring the first exosomes and the second exosomes.

The method for measuring first exosomes and second exosomes in a culture supernatant is not particularly limited, and well-known methods can be used. Examples of methods for measuring first exosomes include an immunochemical measurement method in which an antibody (first antibody) having a specific binding activity for a first exosome membrane protein is used. Examples of methods for measuring second exosomes include an immunochemical measurement method in which an antibody (second antibody) having a specific binding activity for a second exosome membrane protein is used.

A first labeled substance is preferably bound to the first antibody. A second labeled substance is preferably bound to the second antibody. The labeled substances are not particularly limited, and any labeled substances usually used for immunochemical measurement methods can be used without particular limitations. Examples of labeled substances include: solid-phase carrier particles (such as resin beads such as polystyrene and glycidyl methacrylate; magnetic beads; and metal nanoparticles); enzyme labels such as peroxidase (for example, horseradish peroxidase) and alkaline phosphatase; fluorescent labels such as carboxyfluorescein (FAM), 6-carboxy-4',5'-dichloro-2',7'-dimethoxyfluorescein (JOE), fluorescein isothiocyanate (FITC), tetrachlorofluorescein (TET), 5'-hexachloro-fluorescein-CE phosphoramidite (HEX), Cy3, Cy5, Alexa568, and Alexa647; radioisotope labels such as iodine 125; electrochemiluminescence labels such as ruthenium complexes; and biotin. Binding of labeled substances to a first antibody and a second antibody can be performed through a well-known method. The first labeled substance and the second labeled substance are preferably different from each other.

Step (A) may be performed using a third antibody having a specific binding activity for a third exosome membrane protein in addition to the above-described first and second antibodies. The third exosome membrane protein is preferably different from the first exosome membrane protein and the second exosome membrane protein. An exosome membrane protein possessed by at least some first exosomes and at least some second exosomes can be used as the third exosome membrane protein. Examples of third exosome membrane proteins include pan-exosome membrane proteins (CD9, CD63, and CD81). In a case where CD9 is used as a first exosome membrane protein and CD63 is used as a second exosome membrane protein, CD81 can be used as a third exosome membrane protein.

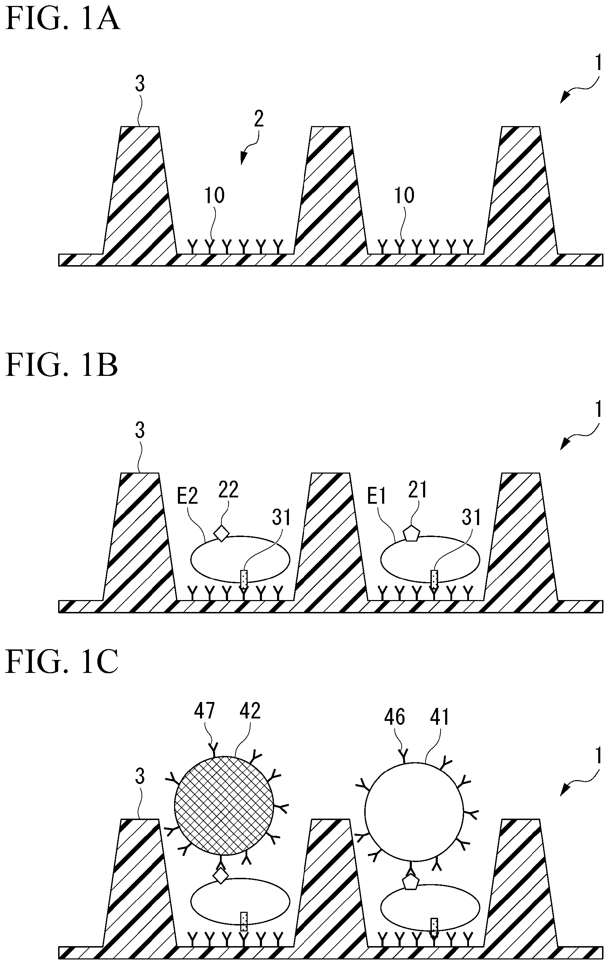

Step (A) may include Step (i) of bringing the culture supernatant into contact with the third antibody immobilized on a solid-phase carrier, Step (ii) of bringing the first antibody and the second antibody into contact with the solid-phase carrier after Step (i), and Step (iii) of measuring the first labeled substance and the second labeled substance captured on the solid-phase carrier after Step (ii). Hereinafter, the case where Step (A) includes the Steps (i) to (iii) will be described with reference to Fig. 1.

«Step (i)»

In Step (i), a culture supernatant is brought into contact with the third antibody immobilized on the solid-phase carrier.

Fig. 1A shows a solid-phase carrier 1 to which third antibodies 10 are immobilized. The solid-phase carrier 1 has concave portions 2 and convex portions 3, and the third antibodies 10 are immobilized on the concave portions 2.

The material of the solid-phase carrier 1 is not particularly limited, and examples thereof include: resins such as polystyrene, polyolefin (such as polyethylene and polypropylene), cycloolefin polymers, and polycarbonates; and glass. The solid-phase carrier 1 may be a well plate which is surface-treated so that the third antibodies 10 can bind thereto, and the concave portion 2 may be a well of the well plate. Alternatively, the solid-phase carrier 1 may be one obtained by providing the concave portions 2 and the convex portions 3 on a surface-treated substrate.

Well-known methods can be performed as the method for immobilizing the third antibodies 10 on the solid-phase carrier 1. For example, the surface on which third antibodies 10 are immobilized may be surface-treated so that proteins are physically adsorbed due to surface hydrophobic interactions or may be surface-treated so as to have an amino group, a carboxyl group, or the like. Alternatively, the third antibodies 10 may be immobilized on the concave portions 2 using an avidin-biotin bond.

In order to bringing a culture supernatant into contact with the third antibodies 10, the culture supernatant may be supplied onto the surface of the solid-phase carrier 1 on which the third antibodies 10 are immobilized.

In a case where exosomes having a third exosome membrane protein 31 is contained in the culture supernatant, exosomes are captured in the concave portions 2 through binding of the third exosome membrane proteins 31 to the third antibodies 10. In Fig. 1B, a first exosome E1 having the third exosome membrane protein 31 and a first exosome membrane protein 21 and a second exosome E2 having the third exosome membrane protein 31 and a second exosome membrane protein 22 are captured in the concave portions 2.

After the culture supernatant is brought into contact with the third antibodies 10, the solid-phase carrier 1 may be appropriately washed using a washing liquid. Exosomes that do not bind to the third antibodies 10 can be removed by washing the solid-phase carrier 1. The washing liquid is not particularly limited, and any washing liquid usually used for immunochemical measurement methods can be used without particular limitation. Examples of washing liquids include, but are not limited to, buffer solutions such as PBS, a tris buffer solution, and an HEPES buffer solution; a mixture obtained by adding a surfactant such as Tween 20 to the buffer solution; and pure water.

The solid-phase carrier 1 may be subjected to a blocking treatment before bringing a culture supernatant into contact with the solid-phase carrier 1. By performing the blocking treatment, it is possible to suppress nonspecific binding to the solid-phase carrier.

The blocking treatment can be performed by bringing a blocking liquid into contact with a solid-phase carrier on which capturing antibodies are immobilized. The blocking liquid is not particularly limited, and any blocking liquid usually used for immunochemical measurement methods can be used without particular limitation. Examples of blocking liquids include a buffer solution containing about 1% to 5% skim milk or bovine serum albumin (BSA). The buffer solution for blocking liquid is not particularly limited, and examples thereof include PBS, PBS-T, a tris buffer solution, and a HEPES buffer solution.

«Step (ii)»

In Step (ii), a first antibody and a second antibody are brought into contact with the solid-phase carrier.

The first labeled substance and the second labeled substance are respectively bound to the first antibody and the second antibody used in Step (ii).

The exosomes (E1 and E2) are captured on the solid-phase carrier 1 after Step (i) via the third antibodies 10. As shown in Fig. 1C, when a first antibody 46 to which a first labeled substance 41 is bound is brought into contact therewith, the first antibody 46 is bound to the first exosome membrane protein 21 of the exosome E1. In addition, when a second antibody 47 to which a second labeled substance 42 is bound is brought into contact therewith, the second antibody 47 is bound to the second exosome membrane protein 22 of the exosome E2.

After the first antibodies 46 and the second antibodies 47 are brought into contact with the solid-phase carrier 1, the solid-phase carrier 1 may be appropriately washed using a washing liquid. First antibodies 46 and second antibodies 47 that do not bind to the exosomes can be removed by washing the solid-phase carrier 1. The washing liquid is not particularly limited, and any washing liquid usually used for immunochemical measurement methods can be used without particular limitation. Examples of washing liquids include, but are not limited to, buffer solutions such as PBS, a tris buffer solution, and an HEPES buffer solution; a mixture obtained by adding a surfactant such as Tween 20 to the buffer solution; and pure water.

<<Step (iii)>>

In Step (iii), the first labeled substance and the second labeled substance captured on the solid-phase carrier are measured.

As shown in Fig. 1C, the first labeled substance 41 is captured on the solid-phase carrier 1 after Step (ii) via the first antibody 46 and the first exosome E1. In addition, the second labeled substance 42 is captured via the second antibody 47 and the second exosome E2. Therefore, it is possible to indirectly measure the first exosome E1 by measuring the first labeled substance 41 captured on the solid-phase carrier 1. In addition, it is possible to indirectly measure the second exosome E2 by measuring the second labeled substance 42 captured on the solid-phase carrier 1.

A method for measuring the first labeled substance 41 and the second labeled substance 42 is not particularly limited, and a well-known method can be used depending on types of labeled substances.

For example, in a case where a labeled substance is an enzyme label, a chromogenic substrate for the enzyme label is added to perform a chromogenic reaction. Subsequently, the absorbance or the like of the reaction solution can be measured after the chromogenic reaction to measure a substance to be detected. For example, a labeled substance is a fluorescent label, the fluorescence intensity can be measured to measure the labeled substance. In a case where a labeled substance is solid-phase carrier particles, the number of solid-phase carrier particles captured on a solid-phase carrier is counted using a particle counter or through microscopic observation and the like to measure the labeled substance.

As the first labeled substance 41 and the second labeled substance 42, ones that can detect signals through the same measurement methods are preferable.

For example, fluorescent labels having different fluorescence wavelengths can be used as the first labeled substance 41 and the second labeled substance 42. In this case, a signal of the first labeled substance 41 and a signal of the second labeled substance 42 can be distinguished due to the difference in fluorescence wavelengths.

For example, enzyme labels having different chromogenic substrates can be used as the first labeled substance 41 and the second labeled substance 42. In this case, a signal of the first labeled substance 41 and a signal of the second labeled substance 42 can be distinguished due to the difference in color tones of color development.

For example, solid-phase carrier particles made of different materials can be used as the first labeled substance 41 and the second labeled substance 42. In this case, a signal of the first labeled substance 41 and a signal of the second labeled substance 42 can be distinguished due to the difference in wavelengths of reflected lights due to laser irradiation.

In a case where the same labeled substance is used as the first labeled substance 41 and the second labeled substance 42, two solid-phase carriers 1 may be prepared and Step (i) and Step (ii) described above may be performed to bring the first antibodies 46 into contact with one solid-phase carrier 1, and to bring the second antibodies 47 into contact with the other solid-phase carrier 1. In this case, since only one of the first labeled substance 41 and the second labeled substance 42 is present on one solid-phase carrier 1, measurement of each labeled substance can be performed.

The method for measuring first exosomes and second exosomes in Step (A) is not particularly limited to the above-described methods, and other well-known immunochemical measurement methods can be used. For example, in a case where fluorescent labels are used as the first labeled substance and the second labeled substance, exosome fractions can be separated from a culture supernatant, first antibodies and second antibodies can be reacted with the exosome fractions, and then the first labeled substance and the second labeled substance can be detected through flow cytometry or the like to measure first exosomes and second exosomes.

(Step (B))

Step (B) is a step of calculating the ratio of second exosomes to first exosomes.

In Step (B), the ratio of the second exosomes to the first exosomes (second exosome/first exosome) may be calculated from the measurement values of the first exosomes and the second exosomes obtained in Step (A).

(Step (C))

Step (C) is a step of determining the condition of cells using the ratio calculated in Step (B) as an index.

The ratio of second exosomes to first exosomes reflects the condition of cells during collecting a culture supernatant. For this reason, the condition of cells during collecting a culture supernatant can be determined using the ratio as an index. For example, a reference range according to the condition of cells can be set in advance and the ratio calculated in Step (B) can be compared with the reference range to determine the condition of cells. More specifically, regarding the ratio of second exosomes to first exosomes, reference ranges for a normal state, a slightly abnormal state, and an abnormal state may be defined, and the condition of cells may be determined depending on which state's reference range the ratio calculated in Step (B) falls within.

The reference values indicating the condition of cells can be set depending on the types of cells and the types of first exosome membrane proteins and second exosome membrane proteins. For example, the same kinds of cells as those to be evaluated are cultured multiple times in advance, the condition of the cells is observed, and the ratio of second exosomes to first exosomes is calculated. The condition of the cells at this time can be confirmed, for example, through microscopic observation. Moreover, a reference range may be set for each condition of the cells.

In addition, in a case where the ratio decreases or increases by more than a predetermined amount from the ratio at the start of culture, it may be determined that cells are in an abnormal state. For example, a relative value when the (second exosome/first exosome) value at the start of culture is 1 is 0.9 or less, 0.8 or less, 0.7 or less, or 0.6 or less, it may be determined that cells are in an abnormal state. Alternatively, a relative value when the (second exosome/first exosome) value at the start of culture is 1 is 1.1 or more, 1.2 or more, 1.3 or more, 1.4 or more, or 1.5 or more, it may be determined that cells are in an abnormal state.

In a case where the first exosome membrane protein is CD9 and the second exosome membrane protein is CD63, the (CD63/CD9) ratio tends to decrease in an abnormal state. For this reason, in a case where the (CD63/CD9) ratio is equal to or less than a threshold value of a predetermined normal state, it can be determined that cells are in an abnormal state. Alternatively, a relative value when the (CD63/CD9) value at the start of culture is 1 is 0.9 or less, 0.8 or less, 0.7 or less, or 0.6 or less, it may be determined that cells are in an abnormal state.

In the evaluation method of the present embodiment, since the condition of cultured cells can be evaluated based on the composition of exosomes in a culture supernatant, it is unnecessary to collect the cultured cells. For this reason, the cultured cells are not wasted, and the cultured cells are not affected by a collection operation. In addition, since the evaluation can be performed through a simple immunochemical measurement method, it is possible to simply evaluate the condition of the cultured cells.

[Second Embodiment]

In one embodiment, the present invention provides a method for evaluating a condition of cells. The method for evaluating a condition of cells of the present embodiment is a method for evaluating a condition of cells, including: Step (a) of measuring first exosomes having a first exosome membrane protein and second exosomes having a second exosome membrane protein in a culture supernatant of the cells at a time (t1); Step (b) of calculating a ratio (t1) of the second exosomes to the first exosomes at the time (t1); Step (c) of measuring the first exosomes and the second exosomes in the culture supernatant of the cells at a time (t2); Step (d) of calculating a ratio (t2) of the second exosomes to the first exosomes at the time (t2); and Step (e) of determining the condition of the cells based on a comparison result of the ratio (t1) and the ratio (t2).

In the evaluation method of the present embodiment, the ratio of second exosomes to first exosomes in a culture supernatant at a time (t1) is compared to the ratio of the second exosomes to the first exosomes in the culture supernatant at a time (t2), and the condition of cells is determined. Accordingly, the change in cultured cells in the same culture system can be evaluated over time.

The same cells, media, culture condition, and the like as those in the first embodiment can be used for the evaluation method of the present embodiment.

(Step (a) and Step (b))

In Step (a), the culture supernatant of the cells may be collected from a culture solution at the time (t1) to measure the first exosomes and the second exosomes in the same manner as in Step (A) of the first embodiment.

In Step (b), the ratio (t1) of the second exosomes to the first exosomes at the time (t1) may be calculated from the measurement values of the first exosomes and the second exosomes obtained in Step (a).

(Step (c) and Step (d))

In Step (c), the culture supernatant of the cells may be collected from a culture solution at the time (t2) to measure the first exosomes and the second exosomes in the same manner as in Step (A) of the first embodiment.

In Step (d), the ratio (t2) of the second exosomes to the first exosomes at the time (t2) may be calculated from the measurement values of the first exosomes and the second exosomes obtained in Step (c).

The time (t2) is a time after the elapse of an arbitrary period of time from the time (t1).

(Step (e))

In Step (e), the ratio (t1) calculated in Step (b) is compared to the ratio (t2) calculated in Step (d), and the condition of the cells is determined based on the comparison result. More specifically, it can be determined whether or not the condition of the cells at the time (t2) has changed compared to the cultured cells at the time (t1).

For example, in a case where the ratio (t2) has changed by more than a predetermined range compared to the ratio (t1), it can be determined that the condition of the cells at the time (t2) has changed from the condition of the cells at the time (t1).

For example, in a case where the change in the ratio (t2) is within a predetermined range compared to the ratio (t1), it can be determined that the condition of the cells at the time (t2) has not changed from the condition of the cells at the time (t1).

The reference values of the change rate of the ratio (t2) with respect to the ratio (t1) which are for determining the change in the condition of cells can be set depending on the types of cells and the types of first exosome membrane proteins and second exosome membrane proteins. For example, the same kinds of cells as those to be evaluated may be cultured multiple times in advance, and whether the ratio of the second exosomes to the first exosomes tends to increase or decrease when the condition of cells is in an abnormal state may be confirmed to set a threshold value when the cells change from a normal state to an abnormal state.

For example, in a case where the ratio of the second exosomes to the first exosomes tends to increase when the cells are in an abnormal state, if the ratio (t2) has increased compared to the ratio (t1), it can be determined that the condition of the cells at the time (t2) has deteriorated compared to the cells at the time (t1). On the other hand, in a case where the ratio (t2) has decreased compared to the ratio (t1), it can be determined that the condition of the cells at the time (t2) has improved compared to the cells at the time (t1).

For example, in a case where the ratio of the second exosomes to the first exosomes tends to decrease when the cells are in an abnormal state, if the ratio (t2) has decreased compared to the ratio (t1), it can be determined that the condition of the cells at the time (t2) has deteriorated compared to the cells at the time (t1). On the other hand, if the ratio (t2) has increased compared to the ratio (t1), it can be determined that the condition of the cells at the time (t2) has improved compared to the cells at the time (t1).

For example, in a case where the first exosome membrane protein is CD9 and the second exosome membrane protein is CD63, the (CD63/CD9) ratio tends to decrease in an abnormal state. For this reason, in a case where the (CD63/CD9) ratio at the time (t2) has decreased compared to the (CD63/CD9) ratio at the time (t1), it can be determined that the condition of the cells at the time (t2) has deteriorated compared to the cells at the time (t1). On the other hand, in a case where the (CD63/CD9) ratio at the time (t2) has increased compared to the (CD63/CD9) ratio at the time (t1), it can be determined that the condition of the cells at the time (t2) has improved compared to the cells at the time (t1). The "(CD63/CD9) ratio" refers to the ratio of the second exosomes having CD63 to the first exosomes having CD9.

According to the evaluation method of the present embodiment, the condition of cultured cells can be confirmed over time by measuring the composition of exosomes in a culture supernatant through an immunochemical method. In the evaluation method of the present embodiment, since it is unnecessary to collect cells using a culture supernatant, the influence of the cell collection on the cultured cells can be avoided.

<Kit for Evaluating Condition of Cells>

In one embodiment, the present invention provides a kit for evaluating a condition of cells. The evaluation kit of the present embodiment includes: a first antibody which has a specific binding activity for a first exosome membrane protein and to which a first labeled substance is bound, a second antibody which has a specific binding activity for a second exosome membrane protein and to which a second labeled substance is bound, and a third antibody which has a specific binding activity for a third exosome membrane protein and is immobilized on a solid-phase carrier. At least some first exosomes having the first exosome membrane protein and at least some second exosomes having the second exosome membrane protein have the third exosome membrane protein.

The kit for evaluating a condition of cells of the present embodiment can be used for carrying out the method for evaluating a condition of cells of the above-described embodiment.

(First Antibody)

A first antibody is an antibody which has a specific binding activity for a first exosome membrane protein and to which a first labeled substance is bound. As the first antibody, the same one as described in the method for evaluating cells in the above-described embodiment can be used. The first antibody is preferably an antibody having a specific binding activity for CD9. Solid-phase carrier particles are preferable as the first labeled substance.

(Second Antibody)

A second antibody is an antibody which has a specific binding activity for a second exosome membrane protein and to which a second labeled substance is bound. As the second antibody, the same one as described in the method for evaluating cells in the above-described embodiment can be used. The second antibody is preferably an antibody having a specific binding activity for CD63. Solid-phase carrier particles are preferable as the second labeled substance. The second labeled substance is preferably different from the first labeled substance.

(Third Antibody)

A third antibody is an antibody which has a specific binding activity for a third exosome membrane protein and is immobilized on a solid-phase carrier. As the third antibody, the same one as described in the method for evaluating cells in the above-described embodiment can be used. The third antibody is preferably an antibody having a specific binding activity for CD81. As the solid-phase carrier, the same one as described in the method for evaluating cells in the above-described embodiment can be used. The solid-phase carrier is preferably one that can be used in an exosome counter equipped with an optical pickup.

(Arbitrary Configuration)

The measurement kit of the present embodiment may include an arbitrary configuration in addition to the above-described configuration. Examples of arbitrary configurations include reagents (for example, diluted solution) for processing a sample, various reagents such as a washing liquid, a blocking liquid, and a buffer solution, and the instruction manual.

As the kit for evaluating a condition of cells of the present embodiment, one that can be used in an exosome counter equipped with an optical pickup is preferably used. The "exosome counter" is a device that can count exosomes by detecting signals of labeled substances directly or indirectly bound to exosomes. For example, ExoCounter (registered trademark) (JVC Kenwood) or the like can be used as a commercially available exosome counter. Hereinafter, specific examples of labeled substances and solid-phase carriers that can be used in the exosome counter will be shown, but are not limited thereto.

<<Specific Examples of Solid-Phase Carriers>>

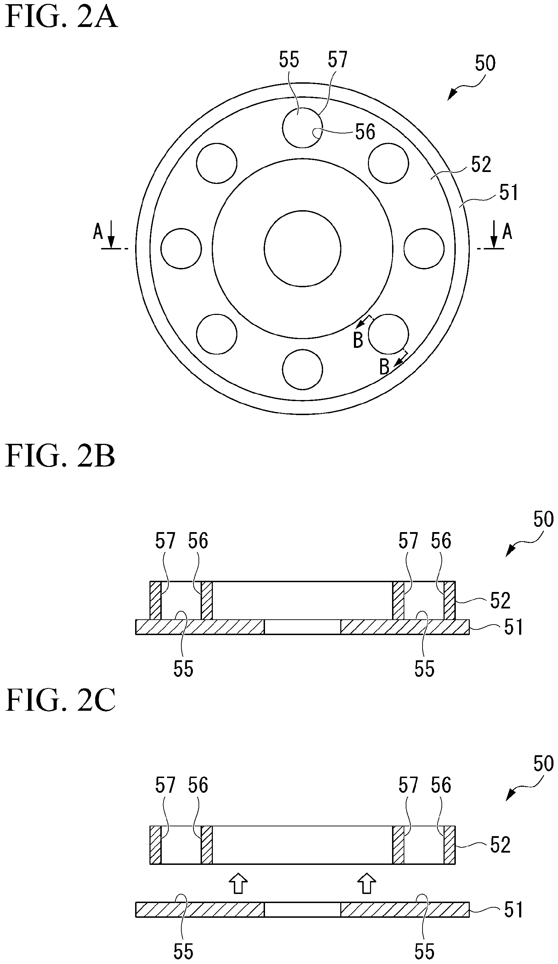

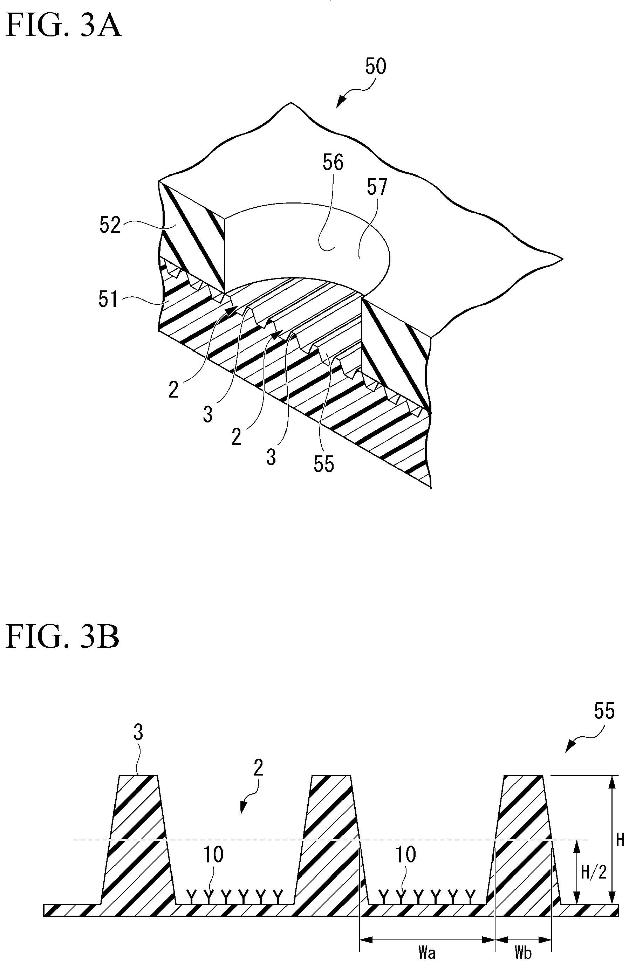

Fig. 2A is a schematic top view of an exosome capture unit 50 containing a solid-phase carrier. Fig. 2B is a schematic cross-sectional view taken along cut line A-A of Fig. 2A. Fig. 2C is a schematic cross-sectional view for illustrating that a cartridge 52 is removable from a substrate 51. Fig. 3A is a partially enlarged perspective view taken along cut line B-B of Fig. 2A.

As shown in Fig. 2A, the exosome capture unit 50 includes the substrate 51 and the cartridge 52.

The substrate 51 has a disk shape equivalent to that of an optical disk such as a Blu-ray disk (BD), DVD, or a compact disk (CD), for example. The substrate 51 is made of, for example, a resin material such as a polycarbonate resin or a cycloolefin polymer generally used in optical disks.

As shown in Fig. 3A, a track region 55 in which convex portions 3 and concave portions 2 are alternately arranged in the radial direction is formed on the surface of the substrate 51. The convex portions 3 and the concave portions 2 are formed in a spiral shape from the inner circumferential portion to the outer circumferential portion. The convex portions 3 correspond to lands of an optical disk. The concave portions 2 correspond to grooves of an optical disk.

The substrate 51 corresponds to the solid-phase carrier 1 of Fig. 1A, and the convex portions 3 and the concave portions 2 formed in the substrate 51 respectively correspond to the convex portions 3 and the concave portions 2 of Fig. 1A. Third antibodies 10 are immobilized on at least the concave portions 2 in the track region 55 in which the concave portions 2 and the convex portions 3 are formed in the substrate 51.

As shown in Fig. 2A, the cartridge 52 has a ring shape. In the cartridge 52, a plurality of cylindrical through-holes 56 are formed in the circumferential direction.

As shown in Figs. 2B and 3A, the exosome capture unit 50 has a plurality of wells 57 formed by the through-holes 56 of the cartridge 52 and the track region 55 of the substrate 51. That is, the inner circumferential surface of the through-holes 56 constitutes the inner peripheral surface of the wells 57, and the track region 55 of the substrate 51 constitutes the bottom surface of the wells 57. The wells 57 are containers for storing sample solutions. In addition, if packing made of an elastically deformable material such as silicone rubber is placed between a through-hole 56 and the substrate 51, the possibility of a solution leaking can be reduced, which is favorable.

As shown in Fig. 2C, the cartridge 52 is detachable from the substrate 51. Detection of exosomes after capture of the exosomes is performed in the substrate 51 alone by removing the cartridge 52 from the substrate 51.

In the exosome capture unit 50, the contact between a culture supernatant and the third antibodies 10 immobilized on the track region 55 in Step (i) can be performed by placing the culture supernatant in the wells 57. In addition, the reaction of the first antibodies 46 and the second antibodies 47 with the exosomes (E1 and E2) bound to the third antibodies 10 in Step (ii) can be performed by placing a solution containing the first antibodies 46 and the second antibodies 47 in the wells 57. In addition, even in a case of performing a washing treatment, a blocking treatment, or the like, these can be performed by placing a washing liquid, a blocking liquid, or the like in the wells 57. Before placing a solution for each treatment in the wells 57, a solution used in the previous treatment is preferably removed from the wells 57.

<<Specific Examples of Solid-Phase Carrier Particles>>

Examples of solid-phase carrier particles include: resin particles such as polystyrene and glycidyl methacrylate; and magnetic beads. In a case where the first labeled substance 41 to which the first antibodies 46 are bound and the second labeled substance 42 to which the second antibodies 47 are bound are used in the same reaction system as shown in Fig. 1C, a solid-phase carrier particle as the first labeled substance 41 and a solid-phase carrier particle as the second labeled substance 42 are preferably made of materials different from each other. Examples of combinations of materials of such solid-phase carrier particles include a combination of resin beads and metal beads. The material of metal beads is most preferably gold or silver, but is not limited thereto. The particle diameter of the solid-phase carrier particles is, for example, preferably 1,000 nm or less, more preferably 1 to 500 nm, still more preferably 10 to 300 nm, and particularly preferably 10 to 200 nm. Magnetic particles are preferably nanoparticles having a nano-sized (1 to 1,000 nm) particle diameter.

Next, the relationship between the dimensions of the concave portions 2 and the convex portions 3 formed on the substrate 51 of the exosome capture unit 50, the dimension of the solid-phase carrier particles, and the average particle diameter of the exosomes will be described.

Fig. 3B is an enlarged cross-sectional view for illustrating the dimensional shapes of the concave portions 2 and convex portions 3 formed in the track region 55 of the substrate 51. The depth of a concave portion 2 (the height of a convex portion 3) is set to H, the width of a concave portion 2 is set to Wa, and the width of a convex portion 3 is set to Wb. The width Wa and the width Wb are widths at the position of H/2 indicated by the dotted line.

The width Wb of a convex portion 3 is preferably smaller than an average particle diameter Ra of exosomes as shown in Inequation (1) described below. By satisfying Inequation (1) described below, it is difficult for exosomes to be positioned on the convex portions 3.

$$\mathrm{Wb} < \mathrm{Ra} \cdots (1)$$

As shown in Inequation (2) described below, the width Wa of a concave portion 2 is preferably larger than the average particle diameter Ra of exosomes and smaller than a value 4 times the average particle diameter Ra. By satisfying Inequation (2) described below, exosomes are likely to be captured in the concave portions 2.

$$\mathrm{Ra} < \mathrm{Wa} < 4 \times \mathrm{Ra} \cdots (2)$$

Exosomes captured in the concave portions 2 generally are deformed from a spherical shape in a direction of expanding the contact area. Assuming that spherical exosomes are deformed into ellipsoids while maintaining their volume, in a case where the diameters of the spheres change by 50%, the diameter of a contact site with a concave portion 2 which is the major axis of a spheroid is increased by about 40%. Furthermore, in reality, exosomes are deformed in a direction in which the area of the contact site is larger than that of the shape of the spheroid. Therefore, the diameter of the contact site is increased by 50% or more, and in some cases, 100% or more of the diameter of the original spherical shape. Accordingly, Wa < 4×Ra of Inequation (2) described above is preferably specified.

The width Wa of a concave portion 2, the width Wb of a convex portion 3, and the particle diameter Rc of a solid-phase carrier particle preferably satisfy Inequation (3) described below.

$$\mathrm{Wb} < \mathrm{Rc} < \mathrm{Wa} < 2 \times \mathrm{Rc} \cdots (3)$$

By satisfying Wb < Rc of Inequation (3) described above, it is difficult for a solid-phase carrier particle to be positioned on the convex portion 3. By satisfying Rc < Wa of Inequation (3) described above, a solid-phase carrier particle is likely to be placed in the concave portion 2. By satisfying Wa < 2×Rc of Inequation (3), it is difficult for two or more solid-phase carrier particles to be placed at the same time in the width direction of the concave portion 2. Therefore, the quantitative relationship between the exosomes and the solid-phase carrier particles can be brought close to 1:1.

As shown in Inequation (4) described below, the particle diameter Rc of a solid-phase carrier particle is preferably larger than the average particle diameter Ra of exosomes.

$$Ra < Rc \cdots (4)$$

By satisfying Inequation (4) described above, it is difficult for a plurality of solid-phase carrier particles to bind to exosomes immobilized on the concave portions 2. Therefore, the quantitative relationship between the exosomes and the solid-phase carrier particles can be brought close to 1:1. By satisfying Inequation (4), the probability that the exosomes and the solid-phase carrier particles reactively meet increases, and as a result, the yield of the reaction can be improved.

As shown in Inequation (5) described below, the depth H of a concave portion 2 is preferably larger than 1/8 times the sum of the average particle diameter Ra of the exosomes and the particle diameter Rc of a solid-phase carrier particle.

$$(Ra + Rc) / 8 < H \cdots (5)$$

By satisfying Inequation (5) described above, exosomes are likely to be captured in the concave portions 2. In addition, since nonspecific adsorption of solid-phase carrier particles on the convex portions 3 is unlikely to occur, the solid-phase carrier particles are likely to bind to the exosomes captured in the concave portions 2.

The depth H of a concave portion 2 more preferably satisfies Inequation (6).

$$(Ra + Rc) / 6 < H \cdots (6)$$

The concave portions 2, the convex portions 3, and the solid-phase carrier particles preferably satisfy all of Inequation (1) to Inequation (5) (or to Inequation (6)), but may only satisfy some of the inequations.

The exosome counter equipped with an optical pickup can irradiate the solid-phase carrier particles captured on the substrate 51 with a laser beam and analyze reflected light thereof to count the number of solid-phase carrier particles captured on the substrate 51. More specifically, the optical pickup irradiates the substrate 51 with a laser beam and receives reflected light from the substrate 51. The optical pickup detects a light reception level of the reflected light, generates light reception level signals JS, and outputs the generated light reception level signals to a control unit equipped with a CPU or the like. The control unit extracts solid-phase carrier particle detection signals KS from the light reception level signals JS output from the optical pickup and counts the solid-phase carrier particle detection signals KS to detect and count the solid-phase carrier particles captured on the substrate 51. Examples of exosome counter having such a mechanism include an analyzer disclosed in Japanese Unexamined Patent Application, First Publication No. 2017-207289.

[Examples]

Hereinafter, the present invention will be described using examples, but is not limited to the following examples.

<Cell Lines>

The following cell lines were used.

Colon cancer cell line: HCT116
Breast cancer cell line: SKBR3
Mesenchymal stem cells: HADSC adipose-derived stem cells

<Media>

The following media were used for culturing cells.

Colon cancer cell line: McCoy's 5A medium + 10% fetal bovine serum
Breast cancer cell line: McCoy's 5A medium + 10% fetal bovine serum
Mesenchymal stem cells: Serum-free medium for stem cells

<Culture>

Approximately $1 \times 10^6$ cells were seeded in 10 mL of a medium and cultured at 37°C in the presence of 5% $CO_2$ until confluent. 50 μL of each culture supernatant was collected after 24 hours, 48 hours, and 72 hours from the confluent state, and CD63-positive exosomes and CD9-positive exosomes were counted. In addition, the cells were observed under a microscope, and the condition of the cells was determined based on determination criteria below based on the form of the cells at the start of culture.

<<Determination Criteria of Condition of Cells>>

Normal: The form of cells has not changed from the start of culture.
Slightly abnormal: There are cells of which the form has changed from the start of culture.
Abnormal: There are many cells of which the form has changed from the start of culture.

<Method for Counting Exosomes>

(Immobilization of CD81 Antibodies on Solid-Phase Carrier)

As CD81 antibodies, exosome monoclonal antibodies Anti-CD81 (Cosmo Bio Co., Ltd.) were used.

Wells provided in a cartridge included in ExoCounter (JVC Kenwood) were used in a solid-phase carrier.

The CD81 antibodies were dissolved in phosphate-buffered saline to have a concentration of 5 μg/mL, and 70 μL thereof was injected into disc wells. Incubation was performed at 37°C for 30 minutes, and the CD81 antibodies were immobilized on the wells through hydrophobic adsorption. After the incubation, the wells were washed with a buffer solution.

(Preparation of Antibody-Bound Nanoparticles)

Exosome monoclonal antibodies Anti-CD63 (Cosmo Bio Co., Ltd.) were used as CD63 antibodies to detect the CD63-positive exosomes. Exosome monoclonal antibodies Anti-CD9 (Cosmo Bio Co., Ltd.) were used as CD9 antibodies to detect the CD9-positive exosomes.

PBS (pH 7.4) containing 400 mM 1-ethyl-3-(3-dimethylaminopropyl) carbodiimide hydrochloride (EDC.HCl) and N-hydroxysuccinimide was added to FG beads (COOH beads; Tamagawa Seiki Co., Ltd.) to cause a reaction at room temperature for 4 hours. After the reaction, the beads were washed with a 50 mM acetate buffer (pH 5.2). Subsequently, an acetate buffer (pH 5.2) containing 1.0 g/L anti-CD9 antibodies or anti-CD63 antibodies was added to the beads, and a reaction was caused at 4°C overnight to immobilize the CD9 antibodies or the CD63 antibodies on the beads. Subsequently, PBS (pH 7.4) containing 0.1 M ethanolamine was added to the beads, and a reaction was caused at 4°C for 5 hours to mask unreacted carboxyl groups. The beads were washed with HEPES buffer (10 mM HEPES, pH 7.9) containing 150 mM KCl, 5 mM EDTA, and 0.1% Tween 20 and preserved at 4°C until these were used. These beads were used as the CD63 antibody-bound nanoparticles and CD9 antibody-bound nanoparticles.

<Counting Exosomes>

50 μL of a culture supernatant was injected into wells on which CD81 antibodies were immobilized, and incubation was performed at 37°C for 2 hours. After the incubation, the wells were washed with a buffer solution. By this operation, CD81-positive exosomes in the culture supernatant were captured on the wells.

Subsequently, CD63 antibody-bound nanoparticles or CD9 antibody-bound nanoparticles were injected into the wells, and incubation was performed at 37°C for 1.5 hours. After the incubation, the wells were washed and the inside of the wells was dried. Subsequently, the wells were separated off from a cartridge, a sample analysis disc was measured with ExoCounter, and the number of nanoparticles bound to the surface of the disc was counted.

<Results>

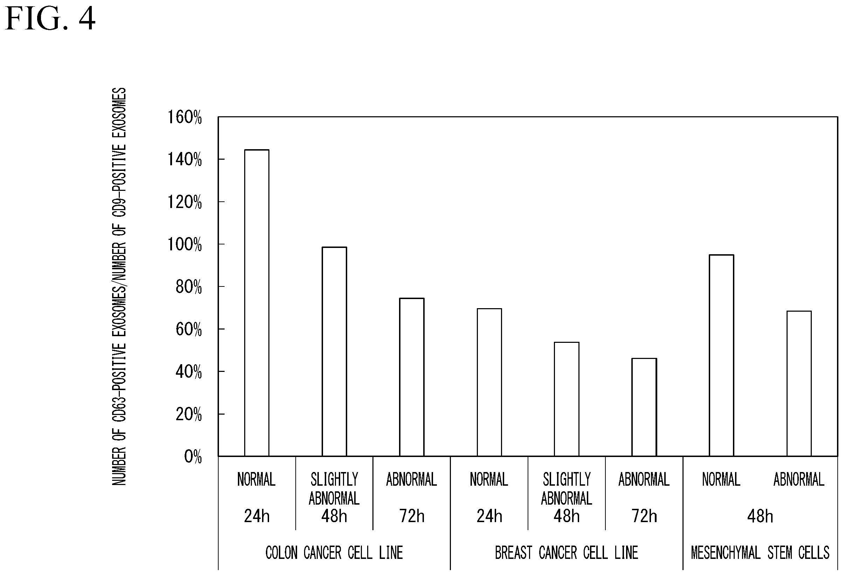

The ratio of the number of CD63-positive exosomes to the number of CD9-positive exosomes (the number of CD63-positive exosomes/the number of CD9-positive exosomes) was calculated from the number of CD63-positive exosomes and the number of CD9-positive exosomes counted in the culture supernatant at each culture time. The results are shown in Fig. 4.

As shown in Fig. 4, if the condition of cells is abnormal in all of the colon cancer cells, the breast cancer cells, and the mesenchymal stem cells, the (number of CD63-positive exosomes/number of CD9-positive exosomes) ratio decreased. It was confirmed from these results that the (number of CD63-positive exosomes/number of CD9-positive exosomes) ratio is used as an index of the condition of cells.

[Industrial Applicability]

According to the present invention, there is provided a method and a kit for evaluating a condition of cells, the method and the kit enabling easy evaluation of the condition of cells using a culture supernatant of the cells.

While the preferred embodiments of the present invention have been described and illustrated above, it should be understood that these are merely examples of the present invention and should not be taken as limiting the present invention. Additions, omissions, substitutions, and other changes can be made without departing from the spirit or the scope of the present invention. Accordingly, the present invention is not limited by the above-described description, and is limited only by the scope of the accompanied claims.

[Reference Signs List]

1 Solid-phase carrier
2 Concave portion
3 Convex portion
10 Third antibodies
21 First exosome membrane protein
22 Second exosome membrane protein
31 Third exosome membrane protein
41 First labeled substance
42 Second labeled substance
46 First antibodies
47 Second antibodies
50 Exosome capture unit
51 Substrate
52 Cartridge
55 Track region
56 Through-holes
57 Wells

**Claims**

1. A method for evaluating a condition of cells, comprising:

   Step (A) of measuring first exosomes having a first exosome membrane protein and second exosomes having a second exosome membrane protein in a culture supernatant of the cells;
   Step (B) of calculating a ratio of the second exosomes to the first exosomes; and
   Step (C) of determining the condition of the cells using the ratio as an index.

2. The method for evaluating a condition of cells according to claim 1,

   wherein Step (A) is a step using

      a first antibody which has a specific binding activity for the first exosome membrane protein and to which a first labeled substance is bound,
      a second antibody which has a specific binding activity for the second exosome membrane protein and to which a second labeled substance is bound, and
      a third antibody which has a specific binding activity for a third exosome membrane protein contained in at least some of the first exosomes and at least some of the second exosomes and is immobilized on a solid-phase carrier, and

   wherein Step (A) comprises

      Step (i) of bringing the culture supernatant into contact with the third antibody immobilized on the solid-phase carrier,
      Step (ii) of bringing the first antibody and the second antibody into contact with the solid-phase carrier after Step (i), and
      Step (iii) of measuring the first labeled substance and the second labeled substance captured on the solid-phase carrier after Step (ii).

3. A method for evaluating a condition of cells, comprising:

   Step (a) of measuring first exosomes having a first exosome membrane protein and second exosomes having a second exosome membrane protein in a culture supernatant of the cells at a time (t1);
   Step (b) of calculating a ratio (t1) of the second exosomes to the first exosomes at the time (t1);
   Step (c) of measuring the first exosomes and the second exosomes in the culture supernatant of the cells at a time (t2);
   Step (d) of calculating a ratio (t2) of the second exosomes to the first exosomes at the time (t2); and
   Step (e) of determining the condition of the cells based on a comparison result of the ratio (t1) and the ratio (t2).

4. The method for evaluating a condition of cells according to claim 3,

   wherein Step (a) and Step (c) are steps using

      a first antibody which has a specific binding activity for the first exosome membrane protein and to which a first labeled substance is bound,
      a second antibody which has a specific binding activity for the second exosome membrane protein and to which a second labeled substance is bound, and
      a third antibody which has a specific binding activity for a third exosome membrane protein contained in at least some of the first exosomes and at least some of the second exosomes and is immobilized on a solid-phase carrier, and

   wherein the Step (a) and Step (c) comprise

      Step (i) of bringing the culture supernatant into contact with the third antibody immobilized on the solid-phase carrier,
      Step (ii) of bringing the first antibody and the second antibody into contact with the solid-phase carrier after Step (i), and
      Step (iii) of measuring the first labeled substance and the second labeled substance captured on the solid-phase carrier after Step (ii).

5. A kit for evaluating a condition of cells, comprising:

   a first antibody which has a specific binding activity for a first exosome membrane protein and to which a first labeled substance is bound,
   a second antibody which has a specific binding activity for a second exosome membrane protein and to which a second labeled substance is bound, and
   a third antibody which has a specific binding activity for a third exosome membrane protein and is immobilized on a solid-phase carrier,
   wherein at least some first exosomes having the first exosome membrane protein and at least some second exosomes having the second exosome membrane protein have the third exo-

some membrane protein.

FIG. 1A

FIG. 1B

FIG. 1C

FIG. 2A

FIG. 2B

FIG. 2C

## FIG. 3A

## FIG. 3B

# FIG. 4

## INTERNATIONAL SEARCH REPORT

International application No.

**PCT/JP2021/033099**

**A.    CLASSIFICATION OF SUBJECT MATTER**

*C12Q 1/06*(2006.01)i; *G01N 33/53*(2006.01)i
FI:   G01N33/53 S; C12Q1/06

According to International Patent Classification (IPC) or to both national classification and IPC

**B.    FIELDS SEARCHED**

Minimum documentation searched (classification system followed by classification symbols)

C12Q1/06; G01N33/53

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | JP 2016-144439 A (ROHTO PHARMACEUTICAL CO., LTD.) 12 August 2016 (2016-08-12) claim 7 | 1-15 |
| A | JP 2019-154320 A (ROHTO PHARMACEUTICAL CO., LTD.) 19 September 2019 (2019-09-19) claim 5 | 1-15 |
| A | WO 2013/094307 A1 (OCHIYA, Takahiro) 27 June 2013 (2013-06-27) claim 1, fig. 1A, | 1-15 |

☐ Further documents are listed in the continuation of Box C.　　☑ See patent family annex.

| | | |
|---|---|---|
| * | Special categories of cited documents: | "T"   later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X"   document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"   document member of the same patent family |

| Date of the actual completion of the international search | Date of mailing of the international search report |
|---|---|
| **16 November 2021** | **30 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2021/033099**

| Patent document cited in search report | | | Publication date (day/month/year) | Patent family member(s) | | | Publication date (day/month/year) |
|---|---|---|---|---|---|---|---|
| JP | 2016-144439 | A | 12 August 2016 | (Family: none) | | | |
| JP | 2019-154320 | A | 19 September 2019 | (Family: none) | | | |
| WO | 2013/094307 | A1 | 27 June 2013 | US | 2015/0017660 | A1 | |
| | | | | claim 1, fig. 1A-C | | | |
| | | | | EP | 2801822 | A1 | |
| | | | | CA | 2860144 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020159896 A
- JP 2018113924 A
- JP 2017207289 A